# EUROPEAN PATENT SPECIFICATION

(11) **EP 4 400 162 B1**
(45) Date of publication and mention of the grant of the patent: **04.11.2026**
(21) Application number: 23174756.9
(22) Date of filing: 23.05.2023
(51) Int. Cl.: A61N 5/10, H05H 3/06

(54) **NEUTRON BEAM SOURCE GENERATION SYSTEM AND NEUTRON BEAM SOURCE GENERATION METHOD**
NEUTRONENSTRAHLQUELLENERZEUGUNGSSYSTEM UND NEUTRONENSTRAHLQUELLENERZEUGUNGSVERFAHREN
SYSTÈME ET PROCÉDÉ DE GÉNÉRATION DE SOURCE DE FAISCEAU DE NEUTRONS

(30) Priority: 13.01.2023 TW 112101492
(43) Date of publication of application: 17.07.2024
(73) Proprietor: Heron Neutron Medical Corp., Zhubei City, Hsinchu County 30261 (TW)
(72) Inventor: HUANG, Kuan-Yan, 30261 Hsinchu County (TW); CHIOU, Shan-Haw, 30261 Hsinchu County (TW); LIN, Chih-Chung, 30261 Hsinchu County (TW); LIOU, Siao-Cing, 30261 Hsinchu County (TW)
(74) Representative: Patentship Patentanwaltsgesellschaft mbH

(56) References cited:
- WO-A1-2022/017193
- US-A1- 2022 062 657
- US-A1- 2023 149 739

## Description

### BACKGROUND OF THE INVENTION

### Field of the Invention

The present disclosure is related to a neutron beam source generation system and a neutron beam source generation method, and, in particular, it is related to a neutron beam source generation system and a neutron beam source generation method with a stable proton beam.

### Description of the Related Art

Neutron capture therapy (NCT) is a type of radiation therapy that is carried out by delivering an element with better neutron absorption to a specific location, then performing a selective treatment by using the difference in the degree of neutron absorption of different elements, thereby precisely destroying cancer cells without destroying other healthy tissues.

Specifically, a neutron beam source for treatment may be formed by generating a proton beam with a particle accelerator and then irradiating a target with the proton beam. However, existing neutron capture therapy devices do not monitor the proton beam in real time, so it is difficult to ensure the stability of the neutron beam source. While existing neutron capture therapy devices have progressively met their intended purposes, they are not entirely satisfactory in every respect. Therefore, there are still some issues to be solved with neutron capture therapy devices.

In particular, US 2022/062657 A1 describes a neutron beam source generation system equipped with a calibration module comprising: a pair of electromagnet components for controlling distribution of the proton beam; a profile-measuring component for measuring the proton beam to obtain a profile distribution; a current-measuring component for measuring the proton beam to obtain a first current value; and a Faraday cup component for optionally shielding the proton beam and measuring the proton beam to obtain a second current value.

### BRIEF SUMMARY OF THE INVENTION

The neutron beam source generation system includes an accelerator, a target, and a calibration module. The accelerator is configured to generate a proton beam that is transmitted in a first direction along a channel. The target is at one end of the channel, wherein a neutron beam source is generated by irradiating the target with the proton beam. The calibration module is disposed in the channel and includes a pair of electromagnet components, a profile-measuring component, a current-measuring component, and a Faraday cup component. The pair of electromagnet components are configured to control distribution of the proton beam in a second direction and in a third direction. The profile-measuring component is configured to measure the proton beam to obtain a profile distribution. The current-measuring component is configured to measure the proton beam to obtain a first current value. The Faraday cup component is configured to optionally shield the proton beam and measure the proton beam to obtain a second current value. The neutron beam source generation method is applied to a neutron beam source generation system, and the neutron beam source generation system includes an accelerator, a target, and a calibration module. The neutron beam source generation method includes the following steps. The accelerator generates a proton beam that is transmitted in a first direction along a channel. The current-measuring component of the calibration module measures the proton beam to obtain a first current value. The Faraday cup component of the calibration module measures the proton beam to obtain a second current value. The profile-measuring component of the calibration module measures the proton beam to obtain the profile distribution. The pair of electromagnet components of the calibration module adjust the distribution of the proton beam in the second direction and in the third direction according to the profile distribution. When the first current value, the second current value, and the profile distribution all meet their preset values, the Faraday cup component is removed so that the proton beam irradiates the target to generate a neutron beam source.
Before removing the Faraday cup component, the neutron beam source generation method further comprises: comparing the first current value and the second current value to obtain a current correspondence table; and after obtaining the neutron counting rate, the neutron beam source generation method further comprises: inferring the second current value from the first current value as measured by the current-measuring component according to the current correspondence table.
The invention is defined in the following claims. Other embodiments, aspects, examples etc. are not a part of the invention and are provided for illustrative purposes only.

### BRIEF DESCRIPTION OF THE DRAWINGS

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion.
FIG. 1 is a block diagram of a neutron beam source generation system according to some embodiments of the present disclosure.
FIG. 2 is a schematic diagram of an accelerator, a calibration module, and a target according to some embodiments of the present disclosure.
FIG. 3 is a schematic diagram of an accelerator, a calibration module, and a target according to other embodiments of the present disclosure.
FIG. 4 is a flowchart of a neutron beam source generation method according to some embodiments of the present disclosure.
FIG. 5 is a flowchart of a current value quality control method according to some embodiments of the present disclosure.
FIG. 6 is a flowchart of a profile quality control method according to some embodiments of the present disclosure.
FIG. 7A and FIG. 7B are a flowchart of a neutron beam source stabilization control method according to some embodiments of the present disclosure.
FIG. 8 is a flowchart of a safety interlock method according to some embodiments of the present disclosure.

### DETAILED DESCRIPTION OF THE INVENTION

The following disclosure provides many different embodiments, or examples, for implementing different features of the provided neutron beam source generation system, neutron beam source stabilization control system, and neutron beam source generation method. Specific examples of features and arrangements are described below to simplify the present disclosure. These are, of course, merely examples and are not intended to be limiting. For example, the formation of a first feature over or on a second feature in the description that follows may include embodiments in which the first and second features are formed in direct contact, and may also include embodiments in which additional features may be formed between the first and second features, such that the first and second features may not be in direct contact. In addition, the present disclosure may repeat reference numerals and/or letters in the various examples. This repetition is for the purpose of simplicity and clarity and does not in itself dictate a relationship between the various embodiments and/or configurations discussed.

The directional terms mentioned herein, such as "up", "down", "left", "right" and similar terms refer to the directions in the drawings. Accordingly, the directional terms used herein are to illustrate, not to limit, the present disclosure.

In some embodiments of the present disclosure, terms about disposing and connecting, such as "disposing", "connecting" and similar terms, unless otherwise specified, may refer to two features are in direct contact with each other, or may also refer to two features are not in direct contact with each other, wherein there is an additional connect feature between the two features. The terms about disposing and connecting may also include the case where both features are movable, or both features are fixed.

In addition, ordinal numbers such as "first", "second", and the like used in the specification and claims are used to modify different features or to distinguish different embodiments or ranges, rather than to limit the number, the upper or lower limits of features, and are not intended to limit the order of manufacture or arrangement of features.

The term "about" used herein generally means within 10%, within 5%, within 3%, within 2%, within 1%, or within 0.5% of a given value or a given range. The value given herein is an approximate value, that is, the meaning of "about" may still be implied without the specific description of "about".

Unless otherwise defined, all terms including technical and scientific terms used herein have the same meaning as commonly understood by a person of ordinary skills in the art. It should be understood that these terms, such as those defined in commonly used dictionaries, should be interpreted as having meanings consistent with the relevant art and the background or context of the present disclosure, and should not be interpreted in an idealized or overly formal manner, unless otherwise defined in the embodiments of the present disclosure.

Some variations of the embodiments are described hereinafter. In different figures and described embodiments, the same or similar reference numerals are used to refer to the same or similar features. It is understood that additional steps may be provided before, during, and after the method, and that some described steps may be replaced or deleted for other embodiments of the method.

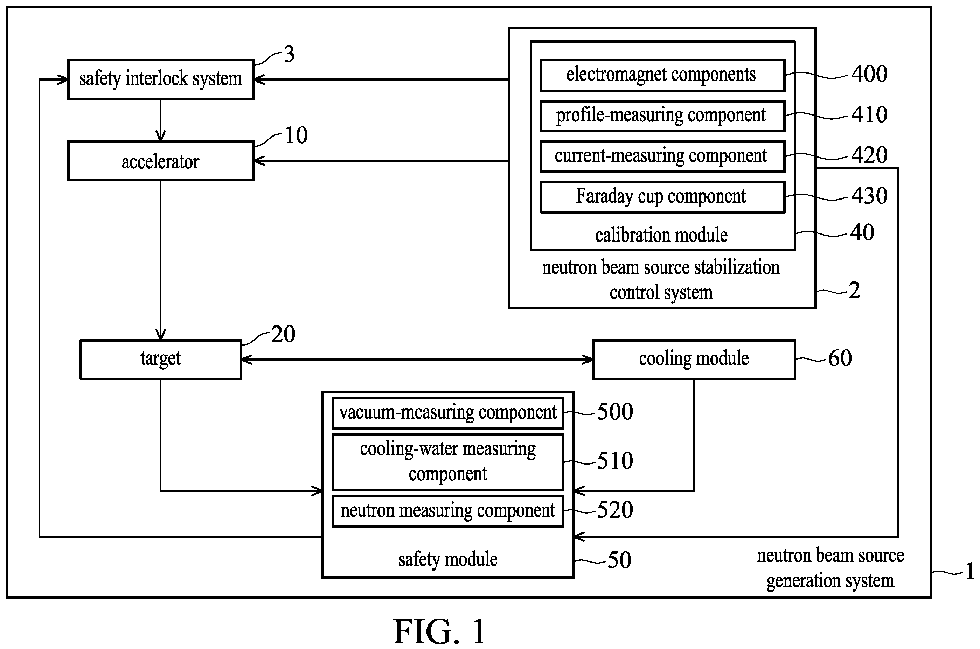

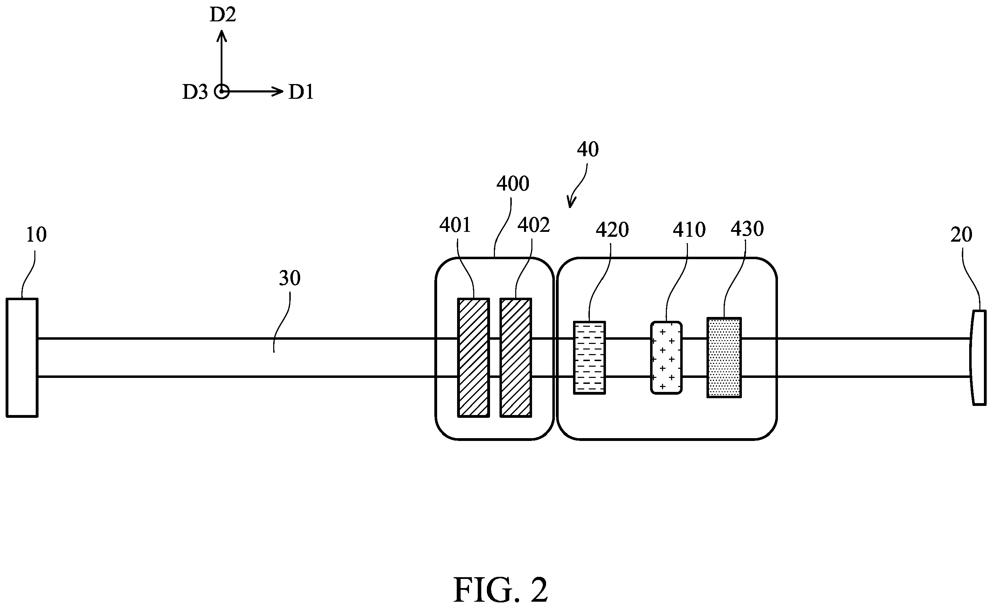

Referring to FIG. 1 and FIG. 2. FIG. 1 is a block diagram of a neutron beam source generation system of the present disclosure, and FIG 2 is a schematic diagram of an accelerator, a calibration module, and a target according to the present disclosure. The neutron beam source generation system 1 includes an accelerator 10, a target 20, and a neutron beam source stabilization control system 2, which may be used to generate a stable neutron beam source. In some embodiments, a neutron beam may be formed by making a neutron beam source generated by the above elements to focus, and then neutron capture therapy may be performed by the neutron beam. That is, the neutron beam source generation system 1 of the present disclosure may be applied in a neutron capture therapy device. However, the present disclosure is not limited thereto. In other embodiments, the neutron beam source generated by the above elements may also be applied to other devices that need to use neutron beams. The detailed structure of the neutron beam source generation system 1 is described hereinafter.

As shown in FIG. 2, the accelerator 10 is configured to generate a proton beam that is transmitted in a first direction D1 toward the target 20 along a channel 30. In some embodiments, the accelerator 10 may be a cyclotron, a linear accelerator, another suitable accelerator, or a combination thereof, but the present disclosure is not limited thereto. In some embodiments, the interior of channel 30 may be in a vacuum state. In some embodiments, the target 20 is at one end of the channel 30. A neutron beam source is generated by irradiating the target 20 with the proton beam. For example, the target 20 may be located within the channel 30, or located outside the channel 30 but connected to and in contact with the channel 30. In some embodiments, the material of the target 20 may be or include lithium (Li), beryllium (Be), other suitable materials, or combinations thereof, but the present disclosure is not limited thereto.

The neutron beam source stabilization control system 2 may include a calibration module 40, disposed between two ends of the channel 30. The proton beam is measured and calibrated by the calibration module 40, so that the proton beam may stably irradiate the target 20, thereby generating a stable neutron beam source.

The calibration module 40 includes a pair of electromagnet components 400 for controlling distribution of the proton beam in a second direction D2 and in a third direction D3. In some embodiments, the pair of electromagnet components 400 may be or include a pair of quadrupole magnets, other suitable electromagnets, or a combination thereof, but the present disclosure is not limited thereto. By adjusting the current input to the quadrupole magnets, the magnetic field of the quadrupole magnets may be changed to focus or defocus the proton beam. Taking FIG. 2 as an example, the pair of electromagnet components 400 may include quadrupole magnets 401 and quadrupole magnets 402. The quadrupole magnets 401 is configured to control the distribution of the proton beam in the second direction D2, and the quadrupole magnets 402 is configured to control the distribution of the proton beam in the third direction D3. The term "distribution" herein may refer to Gaussian distribution, but the present disclosure is not limited thereto. In some embodiments, the first direction D1, the second direction D2, and the third direction D3 are perpendicular to each other, but the present disclosure is not limited thereto. In some embodiments, the first direction D1 is perpendicular to the second direction D2 and the third direction D3, but the second direction D2 is not perpendicular to the third direction D3.

The calibration module 40 further includes a profile-measuring component 410 for measuring the proton beam to obtain a profile distribution. In some embodiments, the profile-measuring component 410 may be or include a beam profiler monitor (BPM), other suitable measuring devices, or a combination thereof, but the present disclosure is not limited thereto. The calibration module 40 controls the distribution of the proton beam in the second direction D2 and in the third direction D3 by the pair of electromagnet components 400 according to the profile distribution as measured by the profile-measuring component 410.

The calibration module 40 further includes a current-measuring component 420 for measuring the proton beam to obtain a first current value. In some embodiments, the current-measuring component 420 may be or include a continuous wave current transformer (CWCT), other suitable measuring devices, or a combination thereof, but the present disclosure is not limited thereto.

The calibration module 40 further includes a Faraday cup (FC) component 430. The Faraday cup component 430 optionally shields the proton beam and measures the proton beam to obtain a second current value. For example, by making the Faraday cup component 430 down to the proton beamline (i.e., placing the Faraday cup component 430 in the path of the proton beam), the proton beam may be shielded, while the proton beam is measured to obtain the second current value. Conversely, by making the Faraday cup component 430 rise off the proton beamline (i.e., the Faraday cup component 430 is moved away from the path of the proton beam), the proton beam may be allowed to pass. The
second current value as measured by the Faraday cup component 430 is more accurate than the first current value as measured by the current-measuring component 420. In other words, calibrating the accelerator 10 with the second current value is more accurate than calibrating the accelerator 10 with the first current value. A plurality of measurements is performed to get a correspondence table between the first current value and the second current value. Then, when the Faraday cup component 430 is risen off the proton beamline, the second current value is inferred from the first current value by the correspondence table, so as to accurately calibrate the accelerator 10 according to the second current value.

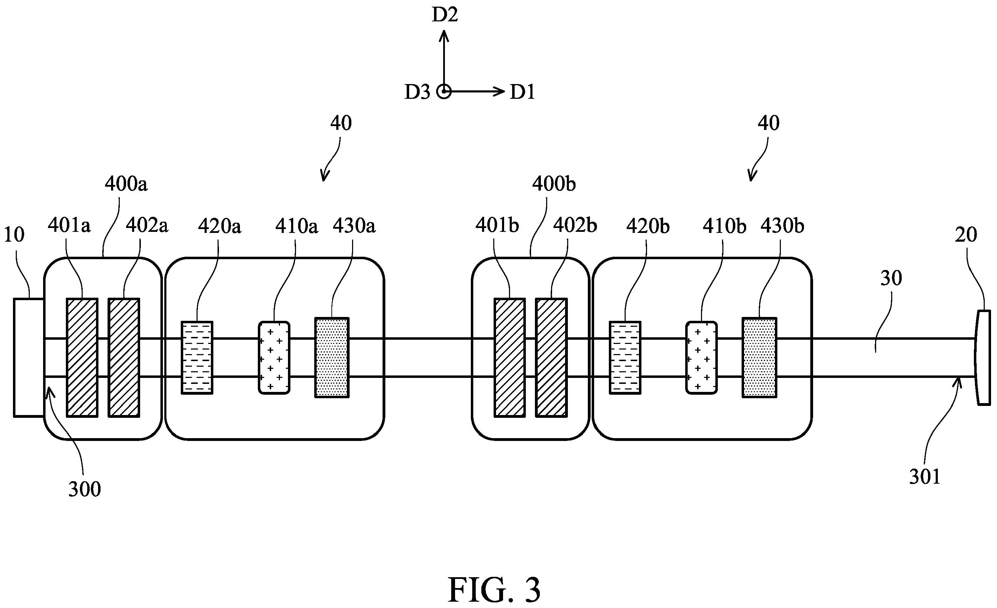

Referring to FIG. 3, which is a schematic diagram of an accelerator, a calibration module, and a target according to other embodiments of the present disclosure. As shown in the figure, the channel 30 has a first side 300 and a second side 301 opposite the first side 300. The accelerator 10 is disposed on the first side 300, and the target 20 is disposed on the second side 301. In other words, the accelerator 10 and the target 20 are respectively located at two ends of the channel 30. In some embodiments, by disposing the same components of the calibration module 40 on the first side 300 of the channel 30 and the second side 301 of the channel 30, the proton beam just leaving the accelerator 10 and the proton beam about to irradiate onto the target 20 may be respectively calibrated. Taking FIG. 3 as an example, the details are as follows.

In some embodiments, the pair of electromagnet components 400 may include a first group of quadrupole magnets 400a and a second group of quadrupole magnets 400b spaced apart from each other. The first group of quadrupole magnets 400a and the second group of quadrupole magnets 400b are configured to control the distribution of the proton beam in the second direction D2 and in the third direction D3. For example, a first group of quadrupole magnets 400a is disposed on the first side 300 of the channel 30 (i.e., the end adjacent to the accelerator 10), and may include a quadrupole magnet 401a and a quadrupole magnet 402a. The second group of quadrupole magnets 400b is disposed on the second side 301 of the channel 30 (i.e., the end adjacent to the target 20), and may include a quadrupole magnet 401b and a quadrupole magnet 402b. The quadrupole magnet 401a and the quadrupole magnet 401b are configured to control the distribution of the proton beam in the second direction D2, and the quadrupole magnet 402a and the quadrupole magnet 402b are configured to control the distribution of the proton beam in the third direction D3. Accordingly, the profile distribution of the proton beam may be calibrated on the first side 300 and the second side 301 of the channel 30 respectively.

Similar to the configuration of the pair of electromagnet components 400, in some embodiments, the profile-measuring component 410 may include a first profile-measuring component 410a and a second profile-measuring component 410b. The current-measuring component 420 includes a first current-measuring component 420a and a second current-measuring component 420b. The Faraday cup component 430 includes a first Faraday cup 430a and a second Faraday cup 430b. For example, the first profile-measuring component 410a, the first current-measuring component 420a, and the first Faraday cup 430a are disposed on the first side 300 of the channel 30 (i.e., the end adjacent to the accelerator 10). The second profile-measuring component 410b, the second current-measuring component 420b, and the second Faraday cup 430b are disposed on the second side 301 of the channel 30 (i.e., the end adjacent to the target 20). Accordingly, the current and profile distribution of the proton beam at the sensing devices on the first side 300 of the channel 30 and the current and profile distribution of the proton beam at the sensing devices on the second side 301 of the channel 30 may be measured respectively. It should be noted that the present disclosure is not limited to the mount positions and numbers of the pair of electromagnet components 400, the profile-measuring component 410, the current-measuring component 420, and the Faraday cup component 430 shown in FIG. 2 and FIG. 3. In some embodiments, the number of each component of the calibration module 40 of the present disclosure may be any positive integer, and may be disposed in different positions in the channel 30 according to requirements.

Still referring to FIG. 1, in some embodiments, the neutron beam source generation system 1 further includes a safety module 50 and a safety interlock system 3. Specifically, the safety module 50 is configured to monitor the operating process of the neutron beam source generation system 1, and the safety interlock system 3 may stop the operation of the neutron beam source generation system 1 or raise an alarm according to the data as measured by the calibration module 40 and the data as measured by the safety module 50 of the neutron beam source stabilization control system 2. By disposing the safety interlock system 3 and the safety module 50, the safety of the neutron beam source generation system 1 may be effectively ensured. In some embodiments, the safety interlock system 3 may include a processor to send instructions to the accelerator 10 or other alarm devices. For example, the processor may include a central processing unit (CPU), a microprocessor (MPU), other suitable processors, or a combination thereof, but the present disclosure is not limited thereto.

In some embodiments, the safety module 50 may include a vacuum-measuring component 500 for measuring the channel 30 to obtain a vacuum value. In some embodiments, the vacuum-measuring component 500 may be or include a resistive gauge, a capacitive gauge, other suitable gauges, or a combination thereof, but the present disclosure is not limited thereto. In some embodiments, when the vacuum value as measured by the vacuum-measuring component 500 deviates from a safe value (for example, the vacuum value is lower than a safe value), the operation of the accelerator 10 may be stopped by the safety interlock system 3.

In some embodiments, the neutron beam source generation system 1 further includes a cooling module 60 for cooling the target 20. In some embodiments where the neutron beam source generation system 1 includes a cooling module 60, the safety module 50 may include a cooling-water measuring component 510. The cooling-water measuring component 510 is configured to measure cooling water in the cooling module 60 to obtain a water temperature value, a water pressure value, a water flow value, and a water resistivity value. In some embodiments, when the water temperature value, the water pressure value, the water flow value, or the water resistivity value as measured by the cooling-water measuring component 510 deviates from a safe value (for example, the water temperature value is higher than a safe value), the operation of the accelerator 10 may be stopped by the safety interlock system 3.

In some embodiments, the safety module 50 may include a neutron measuring component 520 for measuring a neutron beam source to obtain a neutron counting rate. In some embodiments, the neutron measuring component 520 may be or may include Helium - 3 (³He) neutron proportional counters, boron trifluoride (BF₃) neutron proportional counters, other suitable measuring devices, or combinations thereof, but the present disclosure is not limited thereto. In some embodiments, when the neutron counting rate as measured by the neutron measuring component 520 deviates from a safe value (for example, the neutron counting rate is higher than a safe value), the operation of the accelerator 10 may be stopped by the safety interlock system 3.

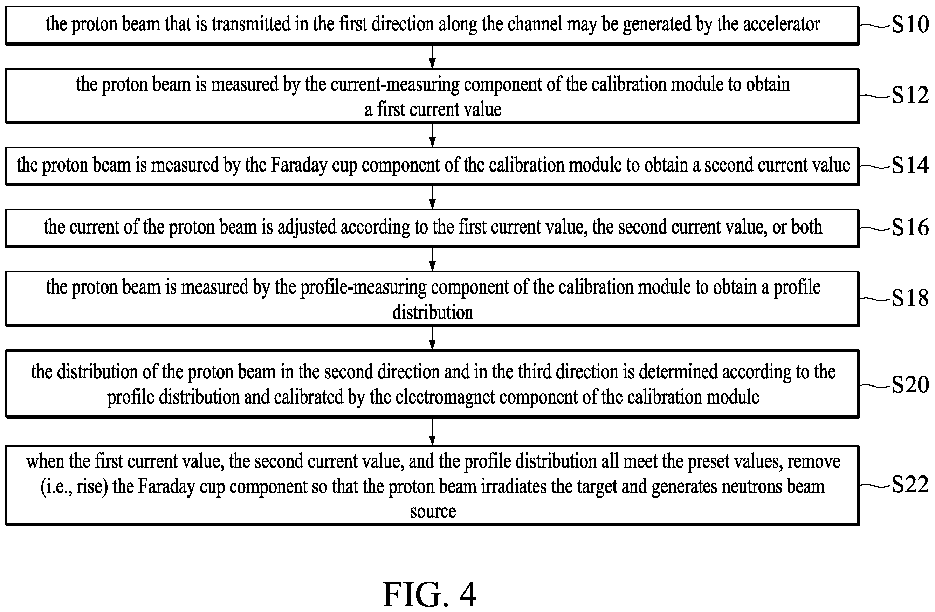

Referring to FIG. 4, which is a flowchart of a neutron beam source generation method of the present disclosure. The neutron beam source generation method includes a neutron beam source stabilization control method for calibrating the proton beam prior to generate the neutron beam source. The neutron beam source stabilization control method includes step S10 to step S22. In step S10, the proton beam that is transmitted in the first direction along the channel may be generated by the accelerator. In step S12, the proton beam is measured by the current-measuring component of the calibration module to obtain the first current value. In step S14, the proton beam is measured by the Faraday cup component of the calibration module to obtain a second current value. After afterstep S12 and step S14, the neutron beam source generation method further includes comparing the first current value and the second current value to obtain a current correspondence table. Since the accuracy of the second current value is higher than that of the first current value, the second current value is inferred from the first current value according to the current correspondence table during the process of generating the neutron beam source (the Faraday cup component is in a off beamline mode), so as to determine the current magnitude of the proton beam more accurately. In step S16, the current of the proton beam is adjusted. For example, the current of the proton beam may be determined according to the first current value or the second current value. In step S18, the proton beam is as measured by the profile-measuring component of the calibration module to obtain a profile distribution. In step S20, the distribution of the proton beam in the second direction and in the third direction is determined according to the profile distribution and calibrated by the pair of electromagnet components of the calibration module. In step S22, when the first current value, the second current value, and the profile distribution all meet the preset values, remove (i.e., rise) the Faraday cup component so that the proton beam irradiates the target and generates neutrons beam source.

In some embodiments, the above step S10 to step S16 may be collectively referred to as a current value quality control method. In some embodiments, step S18 to step S20 may be collectively referred to as a profile quality control method. In some embodiments, the current value quality control method may be performed first, and then the profile quality control method may be performed. Specifically, when the current of the proton beam increases, the space charge force inside the proton beam increases so that the proton beam spreads out (for example, the distribution area increases). Conversely, when the current of the proton beam decreases, the space charge force inside the proton beam decreases so that the proton beam focuses (for example, the distribution area decreases). In other words, the profile distribution of the proton beam will be affected by the magnitude of the current. Therefore, performing the current value quality control method first may prevent the calibrated profile distribution from being affected by changing the magnitude of the current.

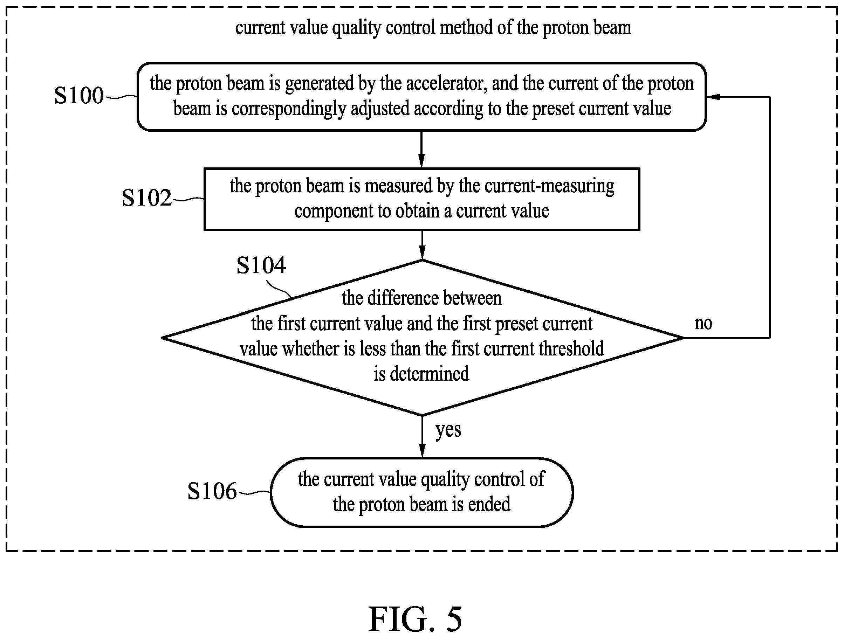

Referring to FIG. 5, which is a flowchart of a current value quality control method according to some embodiments of the present disclosure. In some embodiments, the current value quality control method may be replaced and performed by step S100 to step S106. In step S100, the proton beam is generated by the accelerator, and the current of the proton beam is correspondingly adjusted according to the first preset current value. In step S102, the proton beam is as measured by the current-measuring component to obtain a first current value. In step S104, the difference between the first current value and the first preset current value whether is less than the first current threshold is determined. When the difference between the first current value and the first preset current value is less than the first current threshold, it is determined that the first current value of the proton beam passes the quality control. Conversely, when the difference between the first current value and the first preset current value is greater than or equal to the first current threshold, it is determined that the first current value of the proton beam cannot pass the quality control, and step S100 is performed again. In some embodiments, the first current threshold is 5% of the first preset current value, but the present disclosure is not limited thereto. In other embodiments, the first current threshold may be any value, such as 3%, 10%, 15%, and the like of the first preset current value. In step S106, the current value quality control of the proton beam is ended.

In some embodiments, a Faraday cup component may be used instead of the current-measuring component in step S102. For example, the proton beam may be as measured by a Faraday cup component to obtain the second current value. Next, the difference between the second current value and the second preset current value whether is less than the second current threshold is determined. When the difference between the second current value and the second preset current value is less than the second current threshold, it is determined that the second current of the proton beam passes the quality control. Conversely, when the difference between the second current value and the second preset current value is greater than or equal to the second current threshold, it is determined that the second current of the proton beam cannot pass the quality control, and step S100 is performed again.

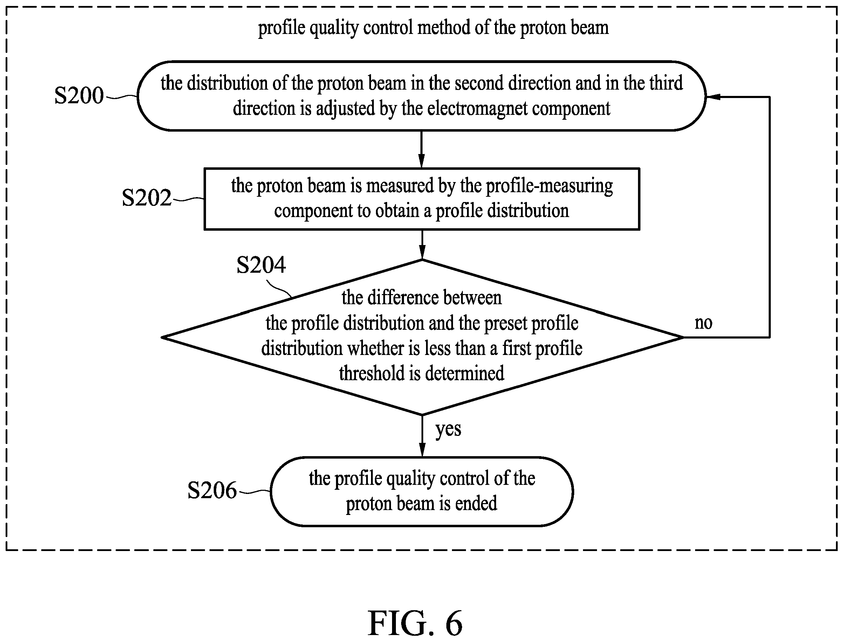

Refer to FIG. 6, which is a flow chart of a profile quality control method according to some embodiments of the present disclosure. In some embodiments, the profile quality control method may be replaced and performed by step S200 to step S206. In step S200, the distribution of the proton beam in the second direction and in the third direction is adjusted by the pair of electromagnet components. In step S202, the proton beam is as measured by the profile-measuring component to obtain a profile distribution. In step S204, the difference between the profile distribution and the preset profile distribution whether is less than a first profile threshold is determined. When the difference between the profile distribution and the preset profile distribution is less than the profile threshold, it is determined that the profile distribution of the proton beam passes the quality control. Conversely, when the difference between the profile distribution and the preset profile distribution is greater than or equal to the profile threshold, it is determined that the profile distribution of the proton beam cannot pass the quality control, and step S200 is performed again. The "difference between the profile distribution and the preset profile distribution" herein may refer to the difference in area between the distribution area and the preset area of the proton beam, but the present disclosure is not limited thereto. In some embodiments, the profile threshold may be 5% of the preset profile distribution. In step S206, the profile quality control of the proton beam is ended.

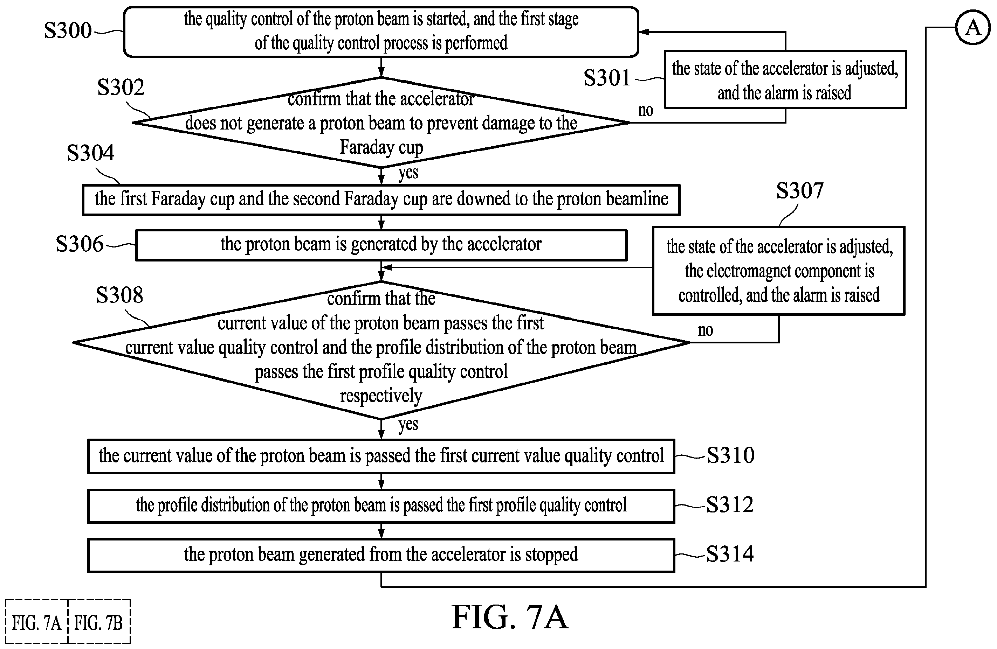

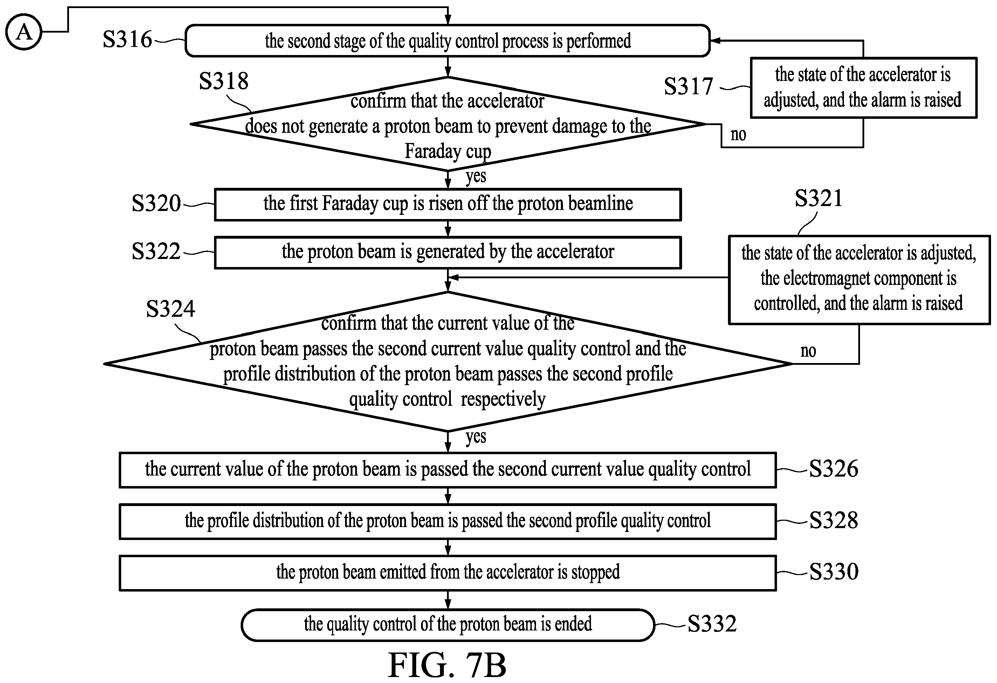

Refer to FIG. 7A and FIG. 7B, which are a flowchart of a neutron beam source stabilization control method according to some embodiments of the present disclosure. In some embodiments, both sides of the channel of the neutron beam source generation system are provided with the pair of electromagnet components, the profile-measuring components, the current-measuring components, and the Faraday cup components (for example, the neutron beam source generation system has a configuration shown in FIG. 3). In this case, the neutron beam source stabilization control method may be replaced and performed by step S300 to step S332, and the steps may be divided into two stages.

In step S300, the quality control of the proton beam is started, and the first stage of the quality control process is performed. In step S302, confirm that the accelerator does not generate a proton beam to prevent damage to the Faraday cup. When the accelerator generates a proton beam, step S301 is performed and step S300 is performed again. In step S301, the state of the accelerator is adjusted (for example, the accelerator is turned off) to stop generating the proton beam, and an alarm is raised by devices such as a speaker and a warning light, but the present disclosure is not limited thereto. In step S304, the first Faraday cup and the second Faraday cup is downed to the proton beamline. In step S306, the proton beam is generated by the accelerator. From step S308 to step S312, whether the current of the proton beam passes the first current value quality control and the profile distribution of the proton beam passes the first profile quality control is respectively confirmed. When the current and/or profile distribution of the proton beam cannot pass the first current value quality control or the first profile quality control, step S307 is performed and step S308 is performed again. In some embodiments, step S307 and step S308 may be similar or identical to the current value quality control method mentioned above (for example, step S10 to step S16, or step S100 to step S106) and profile quality control method (for example, step S18 to step S20, or step S200 to step S206) to perform. In step S314, the proton beam generated from the accelerator is stopped.

In step S316, the second stage of the quality control process is performed. Specifically, in step S316 to step S332, the quality control process similar or identical to that of step S300 to step S314 may be performed again, so the relevant description is omitted. It should be noted that in the first stage of the quality control process (step S300 to step S314), the proton beam is shielded by the first Faraday cup (e.g., step S304). In other words, in the first stage of the quality control process, the main purpose is to measure and control the profile and current of the proton beam between the accelerator and the first Faraday cup. On the other hand, in the second stage of the quality control process (step S316 to step S332), since the first Faraday cup rises, the proton beam is shielded by the second Faraday cup (e.g., step S320). In other words, in the second stage of the quality control process, the main purpose is to measure and control the profile and current of the proton beam between the first Faraday cup and the second Faraday cup. With two times of current value quality control and two times of profile quality control, a more stable proton beam may be produced. In step S332, after the proton beam has passed the two stages of the quality control process, the quality control of the proton beam is ended.

In some embodiments, the neutron beam source generation method further includes a neutron beam source stable generation method for calibrating the proton beam during the process of generating the neutron beam source. For example, after the Faraday cup component is removed, the neutron beam source generation method further includes the following steps. Measuring the proton beam with the current-measuring component to obtain a first current value. Adjusting the current value of the proton beam according to the first current value. Measuring the proton beam with the profile-measuring component to obtain the profile distribution. Adjusting the profile distribution of the proton beam in the second direction and in the third direction according to the profile distribution using the pair of electromagnet components. In other words, some components of the calibration module may be used to calibrate the proton beam before generating the neutron beam source, and may also be used to calibrate the proton beam during the process of generating the neutron beam source.

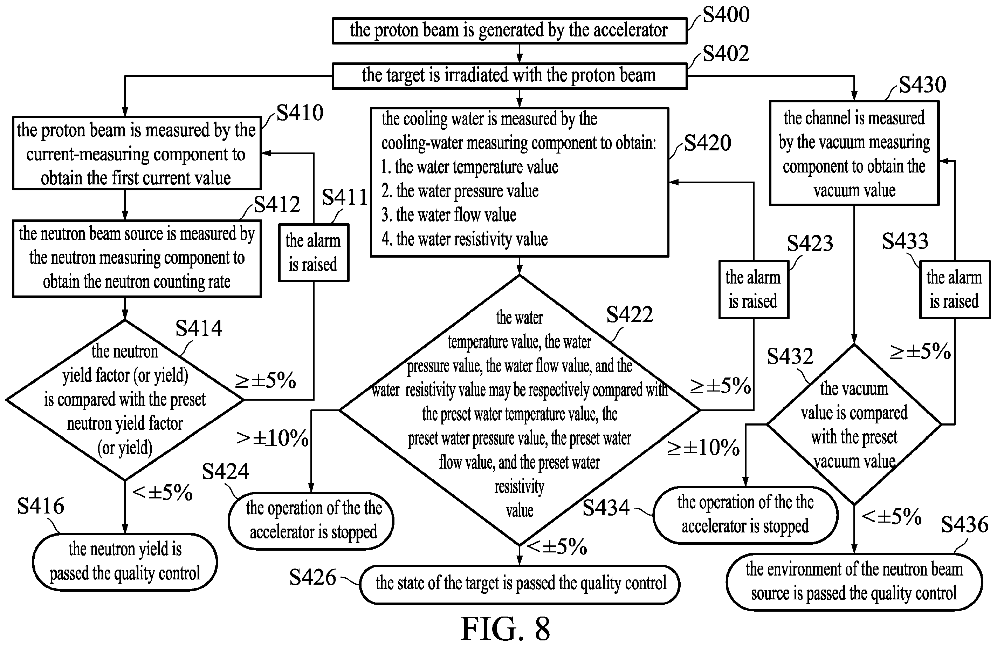

Refer to FIG. 8, which is a flowchart of a safety interlock method according to some embodiments of the present disclosure. In some embodiments, the neutron beam source generation method may further include a safety interlock method for confirming the safety of the system during the process of generating the neutron beam source. In some embodiments, the safety interlock method may include step S400 to step S402, step S410 to step S416, step S420 to step S426, and step S430 to step S436. A detailed description of each step is as follow. In step S400 and step S402, the proton beam may be generated by the accelerator, and the neutron beam source may be generated by irradiating the target with the proton beam.

In some embodiments, step S410 to step S416 are used to confirm whether the yield of the neutron beam source passes the quality control. Specifically, in step S410, the proton beam may be as measured by the current-measuring component of the calibration module to obtain the first current value. In step S412, the neutron beam source may be as measured by the neutron measuring component of the safety module to obtain the neutron counting rate. As used herein, the term "neutron counting rate" refers to the number of neutrons as measured by a device such as a proportional counter. In some embodiments, the number of neutrons may be inferred from the number of pulses as measured by a proportional counter. In step S 414, the neutron counting rate may be divided by the first current value to obtain a first neutron yield factor, and the first neutron yield factor is compared with a first preset neutron yield factor. When the difference between the first neutron yield factor and the first preset neutron yield factor is greater than or equal to the first neutron yield factor threshold, safety interlock control is performed. In some embodiments, the first neutron yield factor threshold may be 5% of the first preset neutron yield factor, but the present disclosure is not limited thereto. In some embodiments, the safety interlock control may include performing step S411 (i.e., raising an alarm) and re-preforming step S410. Alternatively, in step S414, the neutron counting rate may be first converted into a neutron flux, and the neutron flux may be divided by the first current value to obtain the first neutron yield rate. As used herein, the term "neutron flux " refers to the number of neutrons passing by a unit area per unit time. In some embodiments, the term "neutron flux" is equal to the product of neutron density and its average velocity. Next, the first neutron yield may be compared with the first preset neutron yield. When the difference between the first neutron yield and the first preset neutron yield is greater than or equal to a first neutron yield threshold, safety interlock control is performed.

After the step S410, the second current value is inferred from the first current value according to the correspondence table between the first current value and the second current value. Next, in step S414, the neutron counting rate may be divided by the second current value to obtain the second neutron yield factor, and the second neutron yield factor is compared with the second preset neutron yield factor. When the difference between the second neutron yield factor and the second preset neutron yield factor is greater than or equal to the second neutron yield factor threshold, safety interlock control is performed. By using a more accurate value of the second current, the yield of the neutron beam may be estimated more accurately. Alternatively, in some embodiments, the neutron counting rate may be first converted to a neutron flux, and the neutron flux may be divided by the second current value to obtain the second neutron production rate. Then, the second neutron yield may be compared with the second preset neutron yield. When the difference between the second neutron yield and the second preset neutron yield is greater than or equal to the second neutron yield threshold, safety interlock control is performed. In some embodiments, when the difference between the second neutron yield and the second preset neutron yield is greater than or equal to 5%, the safety interlock control may include performing step S411 (i.e., raising an alarm) and re-preforming step S410. In this case, the disposal is determined by professionals.

In some embodiments, step S420 to step S426 are used to confirm whether the state of the target passes the quality control. Specifically, in step S420, the cooling water in the cooling module may be as measured by the cooling-water measuring component of the calibration module to obtain the water temperature value, the water pressure value, the water flow value, and the water resistivity value. In step S422, the water temperature value, the water pressure value, the water flow value, and the water resistivity value may be respectively compared with the preset water temperature value, the preset water pressure value, the preset water flow value, and the preset water resistivity value. When the difference between the water temperature value and the preset water temperature value is greater than or equal to the water temperature value threshold, the difference between the water pressure value and the preset water pressure value is greater than or equal to the water pressure value threshold, the difference between the water flow value and the preset water flow value is greater than or equal to the water flow value threshold, or the difference between the water flow resistivity value and the preset water resistivity value is greater than or equal to the threshold of the water resistivity value, the safety interlock control is performed. In some embodiments, when the difference between any of the above parameters and the corresponding preset parameters is greater than or equal to 5%, the safety interlock control may include performing step S423 (i.e., raising an alarm) and then re-preforming step S420. In this case, the disposal is determined by professionals. In some embodiments, when the difference between any of the above parameters and the corresponding preset parameter is greater than or equal to 10%, the safety interlock control may include performing step S424 to stop the accelerator using the safety interlock system.

In some embodiments, step S430 to step S436 are used to confirm whether the environment of the neutron beam source passes the quality control. Specifically, in step S430, the channel may be as measured by the vacuum-measuring component of the calibration module to obtain the vacuum value. In step S432, the vacuum value may be compared with the preset vacuum value. When the difference between the vacuum value and the preset vacuum value is greater than or equal to the threshold of the vacuum value, safety interlock control is performed. In some embodiments, when the difference between the vacuum value and the preset vacuum value is greater than or equal to 5%, the safety interlock control may include performing step S433 (i.e., raising an alarm) and re-preforming step S430. In this case, the disposal is determined by professionals. In some embodiments, when the difference between the vacuum value and the preset vacuum value is greater than or equal to 10%, the safety interlock control may include performing step S434 to stop the accelerator with the safety interlock system.

It should be noted that, although a difference of 5% or 10% is used herein to determine whether to perform steps such as adjusting the accelerator, raising an alarm, or stopping the accelerator by a safety interlock system, the disclosure is not limited thereto. For example, the difference used to determine in any one of steps S104, S204, S411, S416, S423, and S433 may be replaced to other values, such as 2%, 3%, 4 %, 6 %, 7%, 8%, any range of the above values, or other suitable values. Alternatively, the difference used to determine in any one of steps S424 and S434 may be changed to other values, such as 7%, 8%, 9%, 11%, 12%, 13%, any range of the above values, or other suitable values. In other words, the differences used herein are examples and may be adjusted according to actual needs.

The features between the embodiments of the present disclosure may be arbitrarily combined. In addition, the scope of the present disclosure is not limited thereto the process, machine, manufacturing, material composition, device, method, and step in the specific embodiments described in the specification. A person of ordinary skill in the art will understand current and future process, machine, manufacturing, material composition, device, method, and step from the content disclosed in the present disclosure, as long as the current or future process, machine, manufacturing, material composition, device, method, and step performs substantially the same functions or obtain substantially the same results as the present disclosure. Therefore, the scope of the present disclosure includes the above process, machine, manufacturing, material composition, device, method, and steps. The scope of the present disclosure should be determined by the scope of the claims. It is not necessary for any embodiment or claim of the present disclosure to achieve all of the objects, advantages, and/or features disclosed herein.

The invention is defined in the following claims.

## Claims

1. A neutron beam source generation system (1), comprising:
an accelerator (10) for generating a proton beam that is transmitted in a first direction (D1) along a channel (30);
a target (20) at one end of the channel (30), wherein a neutron beam source is generated by irradiating the target (20) with the proton beam; and
a calibration module (40) disposed in the channel (30), wherein the calibration module (40) comprises:
a pair of electromagnet components (400) for controlling distribution of the proton beam in a second direction (D2) and in a third direction (D3);
a profile-measuring component (410) for measuring the proton beam to obtain a profile distribution;
a current-measuring component (420) for measuring the proton beam to obtain a first current value; and
a Faraday cup component (430) for optionally shielding the proton beam and measuring the proton beam to obtain a second current value, wherein the second current value is used to compare with the first current value to generate a correspondence table,
wherein the calibration module (40) controls the distribution of the proton beam in the second direction (D2) and in the third direction (D3) using the pair of electromagnet components (400) according to the profile distribution, and the calibration module (40) adjusts current of the proton beam according to the second current value that is inferred from the first current value by the correspondence table.

2. The neutron beam source generation system as claimed in claim 1, further comprising a safety interlock system (3) for performing a safety interlock control on the neutron beam source generation system (1) using the calibration module (40) and a safety module (50).

3. The neutron beam source generation system as claimed in claim 2, further comprising a cooling module (60) for cooling the target (20), wherein the safety module (50) is configured to monitor a vacuum state of the channel (30), a state of a cooling water in the cooling module, and an intensity of the neutron beam source.

4. The neutron beam source generation system as claimed in claim 3, wherein the safety module (50) comprises:
a vacuum-measuring component (500) for measuring the channel (30) to obtain a vacuum value;
a cooling-water measuring component (510) for measuring the cooling water in the cooling module (60) to obtain a water temperature value, a water pressure value, a water flow value, and a water resistivity value; and
a neutron measuring component (520) for measuring the neutron beam source to obtain a neutron counting rate.

5. The neutron beam source generation system as claimed in claim 1, wherein the channel (30) has a first side (300) and a second side (301) opposite to the first side (300), the accelerator (10) is disposed on the first side (300), and the target (20) is disposed on the second side (301),
wherein the pair of electromagnet components (400) comprise a first group of quadrupole magnets (400a) and a second group of quadrupole magnets (400b), the profile-measuring component (410) comprises a first profile-measuring component (410a) and a second profile-measuring component (410b), the current-measuring component (420) comprises a first current-measuring component (420a) and a second current-measuring component (420b), and the Faraday cup component (430) comprises a first Faraday cup (430a) and a second Faraday cup (430b),
wherein the accelerator (10), the first group of quadrupole magnets (400a), the first profile-measuring component (410a), the first current-measuring component (420a), and the first Faraday cup (430a) are adjacent to the first side (300) of the channel (30), and the target (20), the second group of quadrupole magnets (400b), the second profile-measuring component (410b), the second current-measuring component (420b), and the second Faraday cup (430b) are adjacent to the second side (301) of the channel (30).

6. The neutron beam source generation system as claimed in claim 1, wherein the pair of electromagnet components (400) comprise a first group of quadrupole magnets (400a) and a second group of quadrupole magnets (400b) spaced apart from each other, and the first group of quadrupole magnets (400a) and the second group of quadrupole magnets (400b) are configured to control the distribution of the proton beam in the second direction (D2) and in the third direction (D3).

7. A neutron beam source generation method applied to a neutron beam source generation system (1), wherein the neutron beam source generation system (1) comprises an accelerator (10), a target (20), and a calibration module (40), and the neutron beam source generation method comprises:
using the accelerator (10) to generate a proton beam that is transmitted in a first direction (D1) along a channel (30);
measuring the proton beam using a current-measuring component (420) of the calibration module (40) to obtain a first current value;
measuring the proton beam using a Faraday cup component (430) of the calibration module (40) to obtain a second current value;
comparing the first current value and the second current value to generate a current correspondence table;
adjusting current of the proton beam according to the second current value;
measuring the proton beam using a profile-measuring component (410) of the calibration module (40) to obtain a profile distribution;
adjusting the distribution of the proton beam in a second direction (D2) and in a third direction (D3) according to the profile distribution using a pair of electromagnet components (400) of the calibration module (40);
when the first current value, the second current value, and the profile distribution all meet preset values, removing the Faraday cup component (430) so that the proton beam irradiates the target (20) to generate a neutron beam source; and
after removing the Faraday cup component (430), adjusting the distribution of the proton beam in the second direction (D2) and in the third direction (D3) according to the second current value that is inferred from the first current value by the correspondence table.

8. The neutron beam source generation method as claimed in claim 7, wherein after removing the Faraday cup component (430), the neutron beam source generation method further comprises:
measuring the proton beam using the current-measuring component (420) to obtain the first current value;
adjusting the current of the proton beam according to the first current value;
measuring the proton beam using the profile-measuring component (410) to obtain the profile distribution; and
adjusting the distribution of the proton beam in the second direction (D2) and in the third direction (D3) using the pair of electromagnet components (400) according to the profile distribution.

9. The neutron beam source generation method as claimed in claim 7, wherein the step of adjusting the distribution of the proton beam in the second direction (D2) and in the third direction (D3) using the pair of electromagnet components (400) of the calibration module (40) comprises:
comparing a preset profile distribution with the profile distribution; and
when a difference between the preset profile distribution and the profile distribution is greater than a profile threshold, adjusting the distribution of the proton beam in the second direction (D2), the third direction (D3), or both.

10. The neutron beam source generation method as claimed in claim 7, wherein the step of adjusting the current of the proton beam according to the first current value, the second current value, or both comprises:
comparing a first preset current value with the first current value; and
when a difference between the first preset current value and the first current value is greater than a first current threshold, adjusting the current of the proton beam.

11. The neutron beam source generation method as claimed in claim 7, wherein the step of adjusting the current of the proton beam according to the first current value, the second current value, or both comprises:
comparing a second preset current value with the second current value; and
when a difference between the second preset current value and the second current value is greater than a second current threshold, adjusting the current of the proton beam.

12. The neutron beam source generation method as claimed in claim 7, wherein the neutron beam source generation system (1) further comprises a cooling module (60) for cooling the target (20), and the neutron beam source generation method further comprises:
using a safety module (50) to monitor a vacuum state of the channel (30), a state of a cooling water in the cooling module (60), and an intensity of the neutron beam source; and
using a safety interlock system (3) to perform a safety interlock control on the neutron beam source generation system (1) according to the current of the proton beam as measured by the calibration module (40) and the vacuum state of the channel (30), the state of the cooling water, and the intensity of the neutron beam source as measured by the safety module (50).

13. The neutron beam source generation method as claimed in claim 12, wherein the step of monitoring the intensity of the neutron beam source using the safety module (50) comprises:
measuring the neutron beam source with a neutron measuring component (520) of the safety module to obtain a neutron counting rate.

14. The neutron beam source generation method as claimed in claim 13, wherein after the step of obtaining the neutron counting rate, the neutron beam source generation method further comprises:
dividing the neutron counting rate by the first current value to obtain a first neutron yield factor;
comparing a first preset neutron yield factor with the first neutron yield factor; and
when a difference between the first preset neutron yield factor and the first neutron yield factor is greater than a first neutron yield factor threshold, stopping the accelerator (10) using the safety interlock system (3).

15. The neutron beam source generation method as claimed in claim 13, wherein before removing the Faraday cup component (430), the neutron beam source
generation method further comprises:
comparing the first current value and the second current value to obtain a current correspondence table; and
after obtaining the neutron counting rate, the neutron beam source generation method further comprises:
inferring the second current value from the first current value as measured by the current-measuring component according to the current correspondence table;
dividing the neutron counting rate by the second current value to obtain a second neutron yield factor;
comparing a second preset neutron yield factor with the second neutron yield factor; and
when a difference between the second preset neutron yield factor and the second neutron yield factor is greater than a second neutron yield factor threshold, stopping the accelerator (10) using the safety interlock system (3).

## Patentansprüche

1. Ein Neutronenstrahlquellenerzeugungssystem (1), umfassend:
einen Beschleuniger (10) zum Erzeugen eines Protonenstrahls, der in einer ersten Richtung (D1) entlang eines Kanals (30) übertragen wird;
ein Target (20) an einem Ende des Kanals (30), wobei eine Neutronenstrahlquelle durch Bestrahlen des Targets (20) mit dem Protonenstrahl erzeugt wird; und
ein Kalibrierungsmodul (40), das in dem Kanal (30) angeordnet ist, wobei das Kalibrierungsmodul (40) umfasst:
ein Paar von Elektromagnetkomponenten (400) zum Steuern einer Verteilung des Protonenstrahls in einer zweiten Richtung (D2) und in einer dritten Richtung (D3);
eine Profilmesseinrichtung (410) zum Messen des Protonenstrahls, um eine Profilverteilung zu erhalten;
eine Strommesseinrichtung (420) zum Messen des Protonenstrahls, um einen ersten Stromwert zu erhalten; und
eine Faraday-Becher-Komponente (430) zum wahlweisen Abschirmen des Protonenstrahls und Messen des Protonenstrahls, um einen zweiten Stromwert zu erhalten, wobei der zweite Stromwert dazu verwendet wird, um mit dem ersten Stromwert verglichen zu werden, um eine Korrespondenztabelle zu erzeugen,
wobei das Kalibrierungsmodul (40) die Verteilung des Protonenstrahls in der zweiten Richtung (D2) und in der dritten Richtung (D3) unter Verwendung des Paars von Elektromagnetkomponenten (400) gemäß der Profilverteilung steuert, und das Kalibrierungsmodul (40) einen Strom des Protonenstrahls gemäß dem zweiten Stromwert einstellt, der aus dem ersten Stromwert durch die Korrespondenztabelle abgeleitet wird.

2. Das Neutronenstrahlquellenerzeugungssystem nach Anspruch 1, ferner umfassend ein Sicherheitsverriegelungssystem (3) zum Durchführen einer Sicherheitsverriegelungssteuerung an dem Neutronenstrahlquellenerzeugungssystem (1) unter Verwendung des Kalibrierungsmoduls (40) und eines Sicherheitsmoduls (50).

3. Das Neutronenstrahlquellenerzeugungssystem nach Anspruch 2, ferner umfassend ein Kühlmodul (60) zum Kühlen des Targets (20),
wobei das Sicherheitsmodul (50) ausgebildet ist, einen Vakuumzustand des Kanals (30), einen Zustand eines Kühlwassers in dem Kühlmodul und eine Intensität der Neutronenstrahlquelle zu überwachen.

4. Das Neutronenstrahlquellenerzeugungssystem nach Anspruch 3, wobei das Sicherheitsmodul (50) umfasst:
eine Vakuummesseinrichtung (500) zum Messen des Kanals (30), um einen Vakuumwert zu erhalten;
eine Kühlwassermesseinrichtung (510) zum Messen des Kühlwassers in dem Kühlmodul (60), um einen Wassertemperaturwert, einen Wasserdruckwert, einen Wasserflusswert und einen Wasserwiderstandswert zu erhalten; und
eine Neutronenmesseinrichtung (520) zum Messen der Neutronenstrahlquelle, um eine Neutronenzählrate zu erhalten.

5. Das Neutronenstrahlquellenerzeugungssystem nach Anspruch 1, wobei der Kanal (30) eine erste Seite (300) und eine der ersten Seite (300) gegenüberliegende zweite Seite (301) aufweist, der Beschleuniger (10) auf der ersten Seite (300) angeordnet ist und das Target (20) auf der zweiten Seite (301) angeordnet ist,
wobei das Paar von Elektromagnetkomponenten (400) eine erste Gruppe von Quadrupolmagneten (400a) und eine zweite Gruppe von Quadrupolmagneten (400b) umfasst, die Profilmesseinrichtung (410) eine erste Profilmesseinrichtung (410a) und eine zweite Profilmesseinrichtung (410b) umfasst, die Strommesseinrichtung (420) eine erste Strommesseinrichtung (420a) und eine zweite Strommesseinrichtung (420b) umfasst und die Faraday-Becher-Komponente (430) einen ersten Faraday-Becher (430a) und einen zweiten Faraday-Becher (430b) umfasst,
wobei der Beschleuniger (10), die erste Gruppe von Quadrupolmagneten (400a), die erste Profilmesseinrichtung (410a), die erste Strommesseinrichtung (420a) und der erste Faraday-Becher (430a) an die erste Seite (300) des Kanals (30) angrenzen, und das Target (20), die zweite Gruppe von Quadrupolmagneten (400b), die zweite Profilmesseinrichtung (410b), die zweite Strommesseinrichtung (420b) und der zweite Faraday-Becher (430b) an die zweite Seite (301) des Kanals (30) angrenzen.

6. Das Neutronenstrahlquellenerzeugungssystem nach Anspruch 1, wobei das Paar von Elektromagnetkomponenten (400) eine erste Gruppe von Quadrupolmagneten (400a) und eine zweite Gruppe von Quadrupolmagneten (400b) umfasst, die voneinander beabstandet sind, und die erste Gruppe von Quadrupolmagneten (400a) und die zweite Gruppe von Quadrupolmagneten (400b) ausgebildet sind, die Verteilung des Protonenstrahls in der zweiten Richtung (D2) und in der dritten Richtung (D3) zu steuern.

7. Ein Neutronenstrahlquellenerzeugungsverfahren, angewendet auf ein Neutronenstrahlquellenerzeugungssystem (1), wobei das Neutronenstrahlquellenerzeugungssystem (1) einen Beschleuniger (10), ein Target (20) und ein Kalibrierungsmodul (40) umfasst, und das Neutronenstrahlquellenerzeugungsverfahren umfasst:
Verwenden des Beschleunigers (10), um einen Protonenstrahl zu erzeugen, der in einer ersten Richtung (D1) entlang eines Kanals (30) übertragen wird;
Messen des Protonenstrahls unter Verwendung einer Strommesseinrichtung (420) des Kalibrierungsmoduls (40), um einen ersten Stromwert zu erhalten;
Messen des Protonenstrahls unter Verwendung einer Faraday-Becher-Komponente (430) des Kalibrierungsmoduls (40), um einen zweiten Stromwert zu erhalten;
Vergleichen des ersten Stromwerts und des zweiten Stromwerts, um eine Stromkorrespondenztabelle zu erzeugen;
Einstellen des Stroms des Protonenstrahls gemäß dem zweiten Stromwert;
Messen des Protonenstrahls unter Verwendung einer Profilmesseinrichtung (410) des Kalibrierungsmoduls (40), um eine Profilverteilung zu erhalten;
Einstellen der Verteilung des Protonenstrahls in einer zweiten Richtung (D2) und in einer dritten Richtung (D3) gemäß der Profilverteilung unter Verwendung eines Paars von Elektromagnetkomponenten (400) des Kalibrierungsmoduls (40);
wenn der erste Stromwert, der zweite Stromwert und die Profilverteilung alle voreingestellte Werte erfüllen, Entfernen der Faraday-Becher-Komponente (430), so dass der Protonenstrahl das Target (20) bestrahlt, um eine Neutronenstrahlquelle zu erzeugen; und
nach dem Entfernen der Faraday-Becher-Komponente (430), Einstellen der Verteilung des Protonenstrahls in der zweiten Richtung (D2) und in der dritten Richtung (D3) gemäß dem zweiten Stromwert, der aus dem ersten Stromwert durch die Korrespondenztabelle abgeleitet wird.

8. Das Neutronenstrahlquellenerzeugungsverfahren nach Anspruch 7, wobei nach dem Entfernen der Faraday-Becher-Komponente (430), das Neutronenstrahlquellenerzeugungsverfahren ferner umfasst:
Messen des Protonenstrahls unter Verwendung der Strommesseinrichtung (420), um den ersten Stromwert zu erhalten;
Einstellen des Stroms des Protonenstrahls gemäß dem ersten Stromwert;
Messen des Protonenstrahls unter Verwendung der Profilmesseinrichtung (410), um die Profilverteilung zu erhalten; und
Einstellen der Verteilung des Protonenstrahls in der zweiten Richtung (D2) und in der dritten Richtung (D3) unter Verwendung des Paars von Elektromagnetkomponenten (400) gemäß der Profilverteilung.

9. Das Neutronenstrahlquellenerzeugungsverfahren nach Anspruch 7, wobei der Schritt des Einstellens der Verteilung des Protonenstrahls in der zweiten Richtung (D2) und in der dritten Richtung (D3) unter Verwendung des Paars von Elektromagnetkomponenten (400) des Kalibrierungsmoduls (40) umfasst:
Vergleichen einer voreingestellten Profilverteilung mit der Profilverteilung; und
wenn eine Differenz zwischen der voreingestellten Profilverteilung und der Profilverteilung größer als ein Profilschwellenwert ist, Einstellen der Verteilung des Protonenstrahls in der zweiten Richtung (D2), der dritten Richtung (D3) oder beiden.

10. Das Neutronenstrahlquellenerzeugungsverfahren nach Anspruch 7, wobei der Schritt des Einstellens des Stroms des Protonenstrahls gemäß dem ersten Stromwert, dem zweiten Stromwert oder beiden umfasst:
Vergleichen eines ersten voreingestellten Stromwerts mit dem ersten Stromwert; und
wenn eine Differenz zwischen dem ersten voreingestellten Stromwert und dem ersten Stromwert größer als ein erster Stromschwellenwert ist, Einstellen des Stroms des Protonenstrahls.

11. Das Neutronenstrahlquellenerzeugungsverfahren nach Anspruch 7, wobei der Schritt des Einstellens des Stroms des Protonenstrahls gemäß dem ersten Stromwert, dem zweiten Stromwert oder beiden umfasst:
Vergleichen eines zweiten voreingestellten Stromwerts mit dem zweiten Stromwert; und
wenn eine Differenz zwischen dem zweiten voreingestellten Stromwert und dem zweiten Stromwert größer als ein zweiter Stromschwellenwert ist, Einstellen des Stroms des Protonenstrahls.

12. Das Neutronenstrahlquellenerzeugungsverfahren nach Anspruch 7, wobei das Neutronenstrahlquellenerzeugungssystem (1) ferner ein Kühlmodul (60) zum Kühlen des Targets (20) umfasst, und das Neutronenstrahlquellenerzeugungsverfahren ferner umfasst:
Verwenden eines Sicherheitsmoduls (50), um einen Vakuumzustand des Kanals (30), einen Zustand eines Kühlwassers in dem Kühlmodul (60) und eine Intensität der Neutronenstrahlquelle zu überwachen; und
Verwenden eines Sicherheitsverriegelungssystems (3), um eine Sicherheitsverriegelungssteuerung an dem Neutronenstrahlquellenerzeugungssystem (1) gemäß dem Strom des Protonenstrahls, wie durch das Kalibrierungsmodul (40) gemessen, und dem Vakuumzustand des Kanals (30), dem Zustand des Kühlwassers und der Intensität der Neutronenstrahlquelle, wie durch das Sicherheitsmodul (50) gemessen, durchzuführen.

13. Das Neutronenstrahlquellenerzeugungsverfahren nach Anspruch 12, wobei der Schritt des Überwachens der Intensität der Neutronenstrahlquelle unter Verwendung des Sicherheitsmoduls (50) umfasst:
Messen der Neutronenstrahlquelle mit einer Neutronenmesseinrichtung (520) des Sicherheitsmoduls, um eine Neutronenzählrate zu erhalten.

14. Das Neutronenstrahlquellenerzeugungsverfahren nach Anspruch 13, wobei das Neutronenstrahlquellenerzeugungsverfahren nach dem Schritt des Erhaltens der Neutronenzählrate ferner umfasst:
Dividieren der Neutronenzählrate durch den ersten Stromwert, um einen ersten Neutronenausbeutefaktor zu erhalten;
Vergleichen eines ersten voreingestellten Neutronenausbeutefaktors mit dem ersten Neutronenausbeutefaktor; und
wenn eine Differenz zwischen dem ersten voreingestellten Neutronenausbeutefaktor und dem ersten Neutronenausbeutefaktor größer als ein erster Neutronenausbeutefaktor-Schwellenwert ist, Stoppen des Beschleunigers (10) unter Verwendung des Sicherheitsverriegelungssystems (3).

15. Das Neutronenstrahlquellenerzeugungsverfahren nach Anspruch 13, wobei das Neutronenstrahlquellenerzeugungsverfahren vor dem Entfernen der Faraday-Becher-Komponente (430) ferner umfasst:
Vergleichen des ersten Stromwerts und des zweiten Stromwerts, um eine Stromkorrespondenztabelle zu erhalten; und
nach dem Erhalten der Neutronenzählrate umfasst das Neutronenstrahlquellenerzeugungsverfahren ferner:
Ableiten des zweiten Stromwerts aus dem ersten Stromwert, wie durch die Strommesseinrichtung gemessen, gemäß der Stromkorrespondenztabelle;
Dividieren der Neutronenzählrate durch den zweiten Stromwert, um einen zweiten Neutronenausbeutefaktor zu erhalten;
Vergleichen eines zweiten voreingestellten Neutronenausbeutefaktors mit dem zweiten Neutronenausbeutefaktor; und
wenn eine Differenz zwischen dem zweiten voreingestellten Neutronenausbeutefaktor und dem zweiten Neutronenausbeutefaktor größer als ein zweiter Neutronenausbeutefaktor-Schwellenwert ist, Stoppen des Beschleunigers (10) unter Verwendung des Sicherheitsverriegelungssystems (3).

## Revendications

1. Système de génération de source de faisceau de neutrons (1), comprenant :
un accélérateur (10) destiné à générer un faisceau de protons qui est transmis dans une première direction (D1) le long d'un canal (30) ;
une cible (20) à une extrémité du canal (30), dans lequel une source de faisceau de neutrons est générée par irradiation de la cible (20) avec le faisceau de protons ; et
un module d'étalonnage (40) disposé dans le canal (30), dans lequel le module d'étalonnage (40) comprend :
une paire de composants électromagnétiques (400) destinée à commander une distribution du faisceau de protons dans une deuxième direction (D2) et dans une troisième direction (D3) ;
un composant de mesure de profil (410) destiné à mesurer le faisceau de protons afin d'obtenir une distribution de profil ;
un composant de mesure de courant (420) destiné à mesurer le faisceau de protons afin d'obtenir une première valeur de courant ; et
un composant à coupelle de Faraday (430) destiné à protéger sélectivement le faisceau de protons et à mesurer le faisceau de protons afin d'obtenir une deuxième valeur de courant, dans lequel la deuxième valeur de courant est utilisée pour être comparée à la première valeur de courant afin de générer une table de correspondance,
dans lequel le module d'étalonnage (40) commande la distribution du faisceau de protons dans la deuxième direction (D2) et dans la troisième direction (D3) en utilisant la paire de composants électromagnétiques (400) conformément à la distribution de profil, et le module d'étalonnage (40) ajuste un courant du faisceau de protons conformément à la deuxième valeur de courant qui est déduite à partir de la première valeur de courant par la table de correspondance.

2. Système de génération de source de faisceau de neutrons selon la revendication 1, comprenant en outre un système de verrouillage de sécurité (3) destiné à effectuer une commande de verrouillage de sécurité sur le système de génération de source de faisceau de neutrons (1) en utilisant le module d'étalonnage (40) et un module de sécurité (50).

3. Système de génération de source de faisceau de neutrons selon la revendication 2, comprenant en outre un module de refroidissement (60) destiné à refroidir la cible (20),
dans lequel le module de sécurité (50) est configuré pour surveiller un état de vide du canal (30), un état d'une eau de refroidissement dans le module de refroidissement, et une intensité de la source de faisceau de neutrons.

4. Système de génération de source de faisceau de neutrons selon la revendication 3, dans lequel le module de sécurité (50) comprend :
un composant de mesure du vide (500) destiné à mesurer le canal (30) afin d'obtenir une valeur de vide ;
un composant de mesure de l'eau de refroidissement (510) destiné à mesurer l'eau de refroidissement dans le module de refroidissement (60) afin d'obtenir une valeur de température de l'eau, une valeur de pression de l'eau, une valeur de débit d'eau et une valeur de résistivité de l'eau ; et
un composant de mesure des neutrons (520) destiné à mesurer la source de faisceau de neutrons afin d'obtenir un taux de comptage des neutrons.

5. Système de génération de source de faisceau de neutrons selon la revendication 1, dans lequel le canal (30) présente un premier côté (300) et un deuxième côté (301) opposé au premier côté (300), l'accélérateur (10) est disposé sur le premier côté (300), et la cible (20) est disposée sur le deuxième côté (301),
dans lequel la paire de composants électromagnétiques (400) comprend un premier groupe d'aimants quadripolaires (400a) et un deuxième groupe d'aimants quadripolaires (400b), le composant de mesure de profil (410) comprend un premier composant de mesure de profil (410a) et un deuxième composant de mesure de profil (410b), le composant de mesure de courant (420) comprend un premier composant de mesure de courant (420a) et un deuxième composant de mesure de courant (420b), et le composant à coupelle de Faraday (430) comprend une première coupelle de Faraday (430a) et une deuxième coupelle de Faraday (430b),
dans lequel l'accélérateur (10), le premier groupe d'aimants quadripolaires (400a), le premier composant de mesure de profil (410a), le premier composant de mesure de courant (420a) et la première coupelle de Faraday (430a) sont adjacents au premier côté (300) du canal (30), et la cible (20), le deuxième groupe d'aimants quadripolaires (400b), le deuxième composant de mesure de profil (410b), le deuxième composant de mesure de courant (420b) et la deuxième coupelle de Faraday (430b) sont adjacents au deuxième côté (301) du canal (30).

6. Système de génération de source de faisceau de neutrons selon la revendication 1, dans lequel la paire de composants électromagnétiques (400) comprend un premier groupe d'aimants quadripolaires (400a) et un deuxième groupe d'aimants quadripolaires (400b) espacés l'un de l'autre, et le premier groupe d'aimants quadripolaires (400a) et le deuxième groupe d'aimants quadripolaires (400b) sont configurés pour commander la distribution du faisceau de protons dans la deuxième direction (D2) et dans la troisième direction (D3).

7. Procédé de génération de source de faisceau de neutrons appliqué à un système de génération de source de faisceau de neutrons (1), dans lequel le système de génération de source de faisceau de neutrons (1) comprend un accélérateur (10), une cible (20) et un module d'étalonnage (40), et le procédé de génération de source de faisceau de neutrons comprend :
l'utilisation de l'accélérateur (10) pour générer un faisceau de protons qui est transmis dans une première direction (D1) le long d'un canal (30) ;
la mesure du faisceau de protons en utilisant un composant de mesure de courant (420) du module d'étalonnage (40) afin d'obtenir une première valeur de courant ;
la mesure du faisceau de protons en utilisant un composant à coupelle de Faraday (430) du module d'étalonnage (40) afin d'obtenir une deuxième valeur de courant ;
la comparaison de la première valeur de courant et de la deuxième valeur de courant afin de générer une table de correspondance de courant ;
l'ajustement du courant du faisceau de protons conformément à la deuxième valeur de courant ;
la mesure du faisceau de protons en utilisant un composant de mesure de profil (410) du module d'étalonnage (40) afin d'obtenir une distribution de profil ;
l'ajustement de la distribution du faisceau de protons dans une deuxième direction (D2) et dans une troisième direction (D3) conformément à la distribution de profil en utilisant une paire de composants électromagnétiques (400) du module d'étalonnage (40) ;
lorsque la première valeur de courant, la deuxième valeur de courant et la distribution de profil satisfont toutes des valeurs prédéfinies, le retrait du composant à coupelle de Faraday (430) de sorte que le faisceau de protons irradie la cible (20) afin de générer une source de faisceau de neutrons ; et
après le retrait du composant à coupelle de Faraday (430), l'ajustement de la distribution du faisceau de protons dans la deuxième direction (D2) et dans la troisième direction (D3) conformément à la deuxième valeur de courant qui est déduite de la première valeur de courant par la table de correspondance.

8. Procédé de génération de source de faisceau de neutrons selon la revendication 7, dans lequel, après le retrait du composant à coupelle de Faraday (430), le procédé de génération de source de faisceau de neutrons comprend en outre :
la mesure du faisceau de protons en utilisant le composant de mesure de courant (420) afin d'obtenir la première valeur de courant ;
l'ajustement du courant du faisceau de protons conformément à la première valeur de courant ;
la mesure du faisceau de protons en utilisant le composant de mesure de profil (410) afin d'obtenir la distribution de profil ; et
l'ajustement de la distribution du faisceau de protons dans la deuxième direction (D2) et dans la troisième direction (D3) en utilisant la paire de composants électromagnétiques (400) conformément à la distribution de profil.

9. Procédé de génération de source de faisceau de neutrons selon la revendication 7, dans lequel l'étape d'ajustement de la distribution du faisceau de protons dans la deuxième direction (D2) et dans la troisième direction (D3) en utilisant la paire de composants électromagnétiques (400) du module d'étalonnage (40) comprend :
la comparaison d'une distribution de profil prédéfinie avec la distribution de profil ; et
lorsqu'une différence entre la distribution de profil prédéfinie et la distribution de profil est supérieure à un seuil de profil, l'ajustement de la distribution du faisceau de protons dans la deuxième direction (D2), la troisième direction (D3), ou les deux.

10. Procédé de génération de source de faisceau de neutrons selon la revendication 7, dans lequel l'étape d'ajustement du courant du faisceau de protons conformément à la première valeur de courant, à la deuxième valeur de courant, ou aux deux, comprend :
la comparaison d'une première valeur de courant prédéfinie avec la première valeur de courant ; et
lorsqu'une différence entre la première valeur de courant prédéfinie et la première valeur de courant est supérieure à un premier seuil de courant, l'ajustement du courant du faisceau de protons.

11. Procédé de génération de source de faisceau de neutrons selon la revendication 7, dans lequel l'étape d'ajustement du courant du faisceau de protons conformément à la première valeur de courant, à la deuxième valeur de courant, ou aux deux, comprend :
la comparaison d'une deuxième valeur de courant prédéfinie avec la deuxième valeur de courant ; et
lorsqu'une différence entre la deuxième valeur de courant prédéfinie et la deuxième valeur de courant est supérieure à un deuxième seuil de courant, l'ajustement du courant du faisceau de protons.

12. Procédé de génération de source de faisceau de neutrons selon la revendication 7, dans lequel le système de génération de source de faisceau de neutrons (1) comprend en outre un module de refroidissement (60) destiné à refroidir la cible (20), et le procédé de génération de source de faisceau de neutrons comprend en outre :
l'utilisation d'un module de sécurité (50) pour surveiller un état de vide du canal (30), un état d'une eau de refroidissement dans le module de refroidissement (60), et une intensité de la source de faisceau de neutrons ; et
l'utilisation d'un système de verrouillage de sécurité (3) pour effectuer une commande de verrouillage de sécurité sur le système de génération de source de faisceau de neutrons (1) conformément au courant du faisceau de protons tel que mesuré par le module d'étalonnage (40), et à l'état de vide du canal (30), à l'état de l'eau de refroidissement et à l'intensité de la source de faisceau de neutrons tels que mesurés par le module de sécurité (50).

13. Procédé de génération de source de faisceau de neutrons selon la revendication 12, dans lequel l'étape de surveillance de l'intensité de la source de faisceau de neutrons en utilisant le module de sécurité (50) comprend :
la mesure de la source de faisceau de neutrons avec un composant de mesure des neutrons (520) du module de sécurité afin d'obtenir un taux de comptage des neutrons.

14. Procédé de génération de source de faisceau de neutrons selon la revendication 13, dans lequel, après l'étape d'obtention du taux de comptage des neutrons, le procédé de génération de source de faisceau de neutrons comprend en outre :
la division du taux de comptage des neutrons par la première valeur de courant afin d'obtenir un premier facteur de rendement neutronique ;
la comparaison d'un premier facteur de rendement neutronique prédéfini avec le premier facteur de rendement neutronique ; et
lorsqu'une différence entre le premier facteur de rendement neutronique prédéfini et le premier facteur de rendement neutronique est supérieure à un premier seuil de facteur de rendement neutronique, l'arrêt de l'accélérateur (10) en utilisant le système de verrouillage de sécurité (3).

15. Procédé de génération de source de faisceau de neutrons selon la revendication 13, dans lequel,
avant le retrait du composant à coupelle de Faraday (430), le procédé de génération de source de faisceau de neutrons comprend en outre :
la comparaison de la première valeur de courant et de la deuxième valeur de courant afin d'obtenir une table de correspondance de courant ; et
après l'obtention du taux de comptage des neutrons, le procédé de génération de source de faisceau de neutrons comprend en outre :
la déduction de la deuxième valeur de courant à partir de la première valeur de courant telle que mesurée par le composant de mesure de courant conformément à la table de correspondance de courant ;
la division du taux de comptage des neutrons par la deuxième valeur de courant afin d'obtenir un deuxième facteur de rendement neutronique ;
la comparaison d'un deuxième facteur de rendement neutronique prédéfini avec le deuxième facteur de rendement neutronique ; et
lorsqu'une différence entre le deuxième facteur de rendement neutronique prédéfini et le deuxième facteur de rendement neutronique est supérieure à un deuxième seuil de facteur de rendement neutronique, l'arrêt de l'accélérateur (10) en utilisant le système de verrouillage de sécurité (3).
